# EUROPÄISCHE PATENTSCHRIFT

(11) **EP 3 638 357 B1**
(45) Veröffentlichungstag und Bekanntmachung des Hinweises auf die Patenterteilung: **05.07.2023**
(21) Anmeldenummer: 18727769.4
(22) Anmeldetag: 24.05.2018
(51) Int. Cl.: A61M 5/178, A61M 5/34, A61M 39/10

(54) **SPRITZE MIT LUER-LOCK-ANSCHLUSS**
SYRINGE WITH A LUER LOCK CONNECTOR
SERINGUE ÉQUIPÉE D'UN EMBOUT LUER-LOCK

(30) Priorität: 12.06.2017 DE 102017112823
(43) Veröffentlichungstag der Anmeldung: 22.04.2020
(73) Patentinhaber: SCHOTT Pharma Schweiz AG, 9000 St. Gallen (CH)
(72) Erfinder: AUERBACH, Judith, 9052 Niederteufen (CH); FISCHER, Bastian, 9000 St. Gallen (CH); DERKSEN, Helena, 9014 St. Gallen (CH); MEISS, Jens, 9000 St. Gallen (CH)
(74) Vertreter: Schott Corporate IP
(86) Internationale Anmeldenummer: PCT/EP2018/063643
(87) Internationale Veröffentlichungsnummer: WO 2018/228788

(56) Entgegenhaltungen:
- EP-A1- 3 020 432
- EP-A2- 0 838 229
- WO-A1-2011/110888
- DE-A1-102014 002 650
- DE-T2- 69 609 881
- DE-U1- 20 316 930
- DE-U1-202009 013 409
- US-A1- 2014 243 797

## Beschreibung

Die Erfindung betrifft eine Spritze mit einem Luer-Lock-Anschluss sowie ein pharmazeutisches Produkt umfassend die Spritze. Spritzenkörper und Luer-Lock-Anschluss der Spritze sind einstückig ausgebildet und bestehen aus einem zyklischem Olefinharz.

Spritzen mit Luer-Lock-Anschluss sind aus dem Stand der Technik bekannt. Über den Luer-Lock-Anschluss kann medizinisches Equipment wie Nadeln, Schläuche oder andere Anschlussteile an die Spritze angeschlossen werden. In der Norm ISO 594/1, 2 und ISO 80369-7 sind die Elemente eines Luer-Lock-Anschlusses genormt, um eine optimale Passung mit hoher Dichtigkeit und Festigkeit sicherzustellen. Ein Luer-Lock-Anschluss umfasst auf der Spritzenseite einen männlichen Luerkonus mit einem mittigen Durchlasskanal für das Medikament, welcher von einem Luerkragen mit einem Innengewinde umgeben ist. Der Konnektor, welcher angeschlossen wird, weist eine weibliches Luerfitting auf, dessen Außenseite beim Einschrauben in das Gewinde des Luerkragens eingreift und so dicht mit dem Luerkonus verbunden wird.

In der Praxis werden viele verschiedene Konnektoren an eine Spritze mit Luer-Lock-Anschluss angeschlossen, da viele Hersteller der Konnektoren eigene, nicht normgerechte Anschlussteile verwenden. So kann die Außenseite eines Konnektors z.B. ein durchgängiges umlaufendes Gewinde entsprechend dem Luerkragen aufweisen, oder aber nur auf einem kurzen axialen Abschnitt mit Elementen versehen sein, welche in das Gewinde des Luerkragens eingreifen.

Es hat sich herausgestellt, dass es auch unter Erfüllung der Anforderungen der ISO 594/1, 2 und ISO 80369-7 zu diversen Problemen an einer Luer-Lock-Verbindung kommen kann, da zyklische Olefinpolymere ein sprödbrüchiges Verhalten aufweisen und bei zu großer, nicht normgerechter Krafteinwirkung durch den Konnektor eine Rissbildung auftreten kann. Dies gilt insbesondere, wenn ein Konnektor mit einem zu großem Drehmoment eingeschraubt wird. Innerhalb des Luer-Lock-Anschlusses können sehr hohe lokale Kräfte auftreten, insbesondere, wenn der Konnektor kein vollständig umlaufendes Außengewinde aufweist. Sowohl im Bereich des inneren Luerkonus als auch im Bereich des äußeren Luerkragens können dann Spannungsrisse auftreten, die zu einem Abscheren des Luerkragens führen können oder sogar in den Spritzenkörper einlaufen können, so dass die Dichtigkeit des Spritzenkörpers oder der Luer-Verbindung gefährdet ist.

Insbesondere bei hochviskosen Medikamenten lastet bei der Entleerung der Spritze auch ein hoher Innendruck auf Spritzenkörper und Luer-Lock-Anschluss. Durch zusätzliche Behandlungen wie z.B. Autoklavieren kann die Stabilität der Verbindung zusätzlich geschwächt werden.

Die US 8,038,182 adressiert ebenfalls das technische Problem der mangelnden Festigkeit eines Luer-Lock-Anschlusses. Sie schlägt vor, an der Außenseite des Luerkonus einen mechanischen Anschlag vorzusehen, welcher die Einschraubtiefe eines Konnektors begrenzt. Ferner wird ein Hohlraum unterhalb des Anschlags vorgeschlagen, in welchen sich der äu-βere Teil des Konnektors, welcher in das Luer-Gewinde eingreift, erstrecken kann. Nachteilig ist, dass der Luerkragen weiterhin bruchgefährdet ist und ein Luer-Lock-Anschluss in der dargestellten Geometrie nur schwer herzustellen ist.

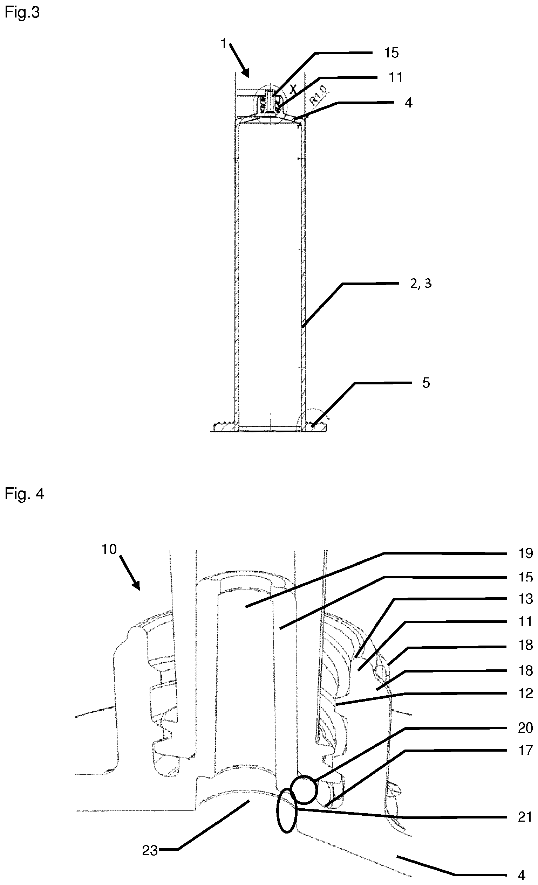

In der EP 0838229 wird vorgeschlagen, einen Anschlag im Innengewinde des Luerkragens vorzusehen, welcher ein zu tiefes Einschrauben eines Konnektors zum Beispiel gemäß Figur 3 der EP 0838229 verhindert. Alternativ wird vorgeschlagen, die Wandstärke zwischen Luer-Lock-Anschluss und Spritzenkörper deutlich anzuheben. Weiter wird vorgeschlagen, eine ringförmige Erhebung bzw. Stufe am Fuß des Luerkragens vorzusehen, welche ein seitliches Ausweichen des weiblichen Konnektors nach außen gemäß Figur 4B verhindern soll. Während die erste Maßnahme ein zu tiefes Einschrauben eines elastomeren Anschlussteils nicht zuverlässig verhindern kann und im Spritzgussverfahren kaum herstellbar ist, kann es bei dem zweiten Lösungsansatz zu Problemen oder erhöhten Kosten bei der Herstellung im Spritzgussverfahren kommen. Der dritte Lösungsansatz ist offensichtlich eng an den besonderen Typ des weiblichen Anschlusses gemäß Figur 4B geknüpft und wird in Verbindung mit elastomeren oder anders geformten Konnektoren eine Belastung des Luerkragens nicht zuverlässig ausschließen können.

DE 10 2014 002650 A1 offenbart einen speziellen Schraubverbinder für medizinische Schlauchsysteme und ein spezielles medizinisches Schlauchsystem mit Schraubverbinder.

DE 203 16 930 U1 befasst sich mit einer speziellen Vorrichtung zum Verbinden und/oder Abdecken bzw. Verschließen von Schläuchen, Behältern oder dergleichen Gegenständen.

DE 696 09 881 T2 offenbart eine spezielle Perforationsvorrichtung und Anschluss für ein enterales Ernährungssystem und ein spezielles Verfahren für das Anschließen.

DE 20 2009 013409 U1 befasst sich mit speziellen Verschlusskappen für Fluidverbindungssysteme.

US 2014/243797 A1 offenbart einen speziellen Fluidleitungsverbinder.

WO 2011/110888 A1 befasst sich mit einer speziellen Arzneimittelabgabevorrichtung.

EP 3 020 432 A1 offenbart eine spezielle Spritze mit einem Fingerflansch und einer Fingerauflage.

Aufgabe der vorliegenden Erfindung ist es, eine Spritze mit einem einstückig ausgebildeten Luer-Lock-Anschluss bereitzustellen, welche mit diversen in der Praxis eingesetzten Konnektoren eine dauerhaft dichte und stabile Luer-Verbindung sicherstellt und sich im Spritzgussverfahren herstellen lässt.

Die Aufgabe wird gelöst durch den unabhängigen Anspruch. Bevorzugte Ausführungsformen sind in den abhängigen Ansprüchen ausgeführt.

Die erfindungsgemäße Spritze umfasst einen Spritzenkörper, welcher einen zylindrischen Abschnitt und einen daran anschließenden kegelstumpfförmigen Abschnitt aufweist, und einen Luer-Lock-Anschluss, welcher einen Luerkragen mit einem Innengewinde und einen Luerkonus mit einem Durchlasskanal aufweist, wobei zwischen Luerkragen und Luerkonus eine Bodenfläche ausgebildet ist, wobei Spritzenkörper und Luer-Lock-Anschluss einstückig ausgebildet sind und aus einem zyklischen Olefinharz bestehen; wobei der Luerkragen auf seiner Außenseite axial verlaufende Verstärkungsrippen aufweist; und wobei die Verstärkungsrippen an ihrer dem kegelstumpfförmigen Abschnitt zugewandten Basis eine radiale Ausdehnung zwischen 0,5 mm und 2,5 mm aufweisen und eine laterale Ausdehnung zwischen 0,5 mm und 2,0 mm aufweisen; und/oder wobei der Übergang der Verstärkungsrippen zum Luerkragen und/oder zum kegelstumpfförmigen Abschnitt verrundet ausgebildet ist mit einem Radius von mindestens 0,3 mm. Überraschenderweise hat sich herausgestellt, dass durch die Verstärkungsrippen am Luerkonus nicht nur die Steifigkeit des Luerkragens deutlich angehoben werden kann, sondern dass durch die Verstärkungsrippen auch die Ausbreitung von Spannungsrissen vom Spritzenkörper abgelenkt werden kann. So breiten sich Risse in zylindrisch ausgebildeten Luerkragen gemäß Stand der Technik ohne Verstärkungsrippen relativ ungestört aus, so dass es zu einem Abscheren bzw. einer Abtrennung des gesamten Luerkragens kommen kann, während Risse in Luerkragen mit Verstärkungsrippen überraschenderweise an den Verstärkungsrippen auslaufen. Auch eine Anhebung der Wandstärke des Luerkragens hat keine vergleichbare Wirkung zu den Verstärkungsrippen gezeigt. Entgegen der Vermutung, dass durch die Rippen die Rissbildung verlangsamt wird, wurde auch beobachtet, dass die Anzahl der Risse durch die Verstärkungsrippen reduziert werden kann.

Der Spritzenkörper dient der Aufnahme des Pharmazeutikums und ist dafür ausgebildet, mit einem Stopfen zusammenwirken. Er besteht zumindest großteils und bevorzugt vollständig aus einem zyklischen Olefinharz wie Cycloolefincopolymer (COC) oder Cycloolefinpolymer (COP). Handelsnamen dieser zyklischen Olefinharze sind beispielsweise Zeonor^{©}, Zeonex^{©} und ARTON^{©}.

Der zylindrische Abschnitt des Spritzenkörpers dient der Aufnahme des Pharmazeutikums und ist dafür ausgebildet, mit einem Stopfen zusammenwirken. Die Innenoberfläche des zylindrischen Abschnitts des Spritzenkörpers kann eine Gleitschicht aufweisen, welche eine geringe Haft- und Gleitreibung in Verbindung mit einem Stopfen sicherstellt. Außen- und / oder Innenseite können auch mit Barriereschichten versehen sein, welche z.B. die Diffusion von Sauerstoff verringern. Die Wandstärke des Spritzenkörpers liegt typischerweise in einem Bereich von 0,7 mm bis 2,5 mm.

Der kegelstumpfförmige Abschnitt des Spritzenkörpers stellt den Bereich des Spritzenkörpers dar, welcher den zylindrischen Abschnitt des Spritzenkörpers mit dem Luer-Lock-Anschluss und der Durchlassöffnung verbindet. Er ist im Allgemeinen kegelförmig ausgebildet, kann aber auch plan ausgebildet sein. Auf dem kegelstumpfförmigen Abschnitt des Spritzenkörpers sind der Luerkragen und der Luerkonus ausgebildet.

Der Luerkragen besteht aus einem rohrförmigen Abschnitt mit einem Innengewinde auf seiner Innenseite und Verstärkungsrippen auf der Außenseite. Außen- und/oder Innenfläche des Luerkragens können einen Winkel zur Längsachse der Spritze von 0° - 2° aufweisen, so dass die Wandstärke zum freien Ende hin geringfügig abnimmt. Dadurch kann eine gute Entformbarkeit des Spritzenkörpers bei einer Herstellung im Spritzgussverfahren erreicht werden, d.h. der Spritzenkörper lässt sich nach dem Öffnen der Spritzgussform gut aus der Form herauslösen.

Das Innengewinde des Luerkragens kann gemäß der Norm ISO 594/2 und ISO 80369-7 ausgebildet sein. Zum Beispiel kann es sich um ein zweiläufiges rechtsdrehendes Gewinde handeln. Die Steigung des Gewindes kann gemäß der Norm ISO 594/2 5 mm betragen, oder zwischen 2 mm und 5 mm.

Der Luerkonus ist bevorzugt gemäß der Norm ISO 594/1 ausgebildet.

Spritzenkörper und Luer-Lock-Anschluss sind einstückig ausgebildet. Unter einstückig ist hierbei zu verstehen, dass der Spritzenkörper und Luer-Lock-Anschluss materialschlüssig verbunden sind und im Wesentlichen aus demselben Grundmaterial bestehen. Das kann beispielsweise durch eine Herstellung der Spritze im Spritzgussverfahren erreicht werden. Ebenso ist aber denkbar ein Anschweißen oder Anformen des Luer-Lock-Anschlusses an einen Spritzenkörper.

In einer bevorzugten Ausführungsform weist der Luerkragen zwischen 4 und 24, bevorzugt zwischen 8 und 16 Verstärkungsrippen auf. Die Zahl der Verstärkungsrippen ist im Allgemeinen so groß, dass eine gleichmäßige Festigkeit des Luerkragens in allen Richtungen erreicht wird, eine einzelne Verstärkungsrippe aber eine ausreichende Stärke aufweisen kann. Besonders bevorzugt weist der Luerkragen 12 Verstärkungsrippen derselben Abmessungen auf, welche in gleichmäßigem Winkelabstand um den Luerkragen angeordnet sind. Die Verstärkungsrippen sind bevorzugt alle gleich groß ausgebildet und in konstanten Winkelschritten um den Luerkragen angeordnet. Ebenso können aber auch mehrere Typen von Verstärkungsrippen mit unterschiedlichen Ausführungen vorgesehen sein.

Gemäß der Erfindung weisen die Verstärkungsrippen an ihrer Basis eine radiale Ausdehnung zwischen 0,5 mm und 2,5 mm auf und weisen eine laterale Ausdehnung zwischen 0,5 mm und 2,0 mm auf; und/oder der Übergang der Verstärkungsrippen zum Luerkragen und/oder zum kegelstumpfförmigen Abschnitt ist verrundet ausgebildet mit einem Radius von mindestens 0,3 mm.

In einer bevorzugten Ausführungsform weisen die Verstärkungsrippen an ihrer Basis eine radiale Ausdehnung zwischen 0,5 mm und 2,5 mm und eine laterale Ausdehnung zwischen 0,5 mm und 2,0 mm auf. Unter der Basis einer Verstärkungsrippe ist hierbei der dem kegelstumpfförmigen Abschnitt zugewandte Bereich einer Verstärkungsrippe zu verstehen. Zum freien Ende des Luerkragens hin können sich die Verstärkungsrippen verjüngen.

In einer bevorzugten Ausführungsform erstrecken sich die Verstärkungsrippen vom kegelstumpfförmigen Abschnitt ausgehend axial bis mindestens zur halben Höhe des Luerkragens und bevorzugt bis zur Oberkante des Luerkragens. Die Verstärkungsrippen weisen also bevorzugt eine Länge auf, die 50 % bis 100 % der Länge des Luerkragens entspricht.

In einer bevorzugten Ausführungsform ist der Übergang der Verstärkungsrippen zum Luerkragen und/oder zum kegelstumpfförmigen Abschnitt verrundet ausgebildet mit einem Radius von mindestens 0,3 mm und bevorzugt 0,5 mm bis 1,5 mm. Durch die verrundeten Übergänge können Lastspitzen vermieden und auftretende Kräfte gleichmäßiger verteilt werden.

In einer bevorzugten Ausführungsform ist auf der Bodenfläche zwischen Luerkragen und Luerkonus und an der Außenseite des Luerkonus eine ringförmige Erhebung angeordnet, welche so ausgebildet ist, dass sie als Anschlag für einen einschraubbaren Konnektor wirkt. Die ringförmige Erhebung kann gemessen vom tiefsten Punkt der Bodenfläche eine Höhe von 0,3 mm bis 2 mm aufweisen und eine radiale Ausdehnung von 0,3 mm bis 1,5 mm. Beim Einschrauben eines Konnektors, welcher am Luerkonus anliegt wird dieser an der ringförmigen Erhebung anstoßen. Weiter bevorzugt weist die ringförmige Erhebung eine plane Oberfläche auf.

In einer weiteren Ausführungsform weist die Spritze eine ringförmigen Aussparung auf, welche radial in der Verlängerung des kegelstumpfförmigen Abschnittes und axial unterhalb der Wandung des Spritzenkonus liegt, so dass zwischen Durchlasskanal und kegelstumpfförmigen Abschnitt ein zylinderförmiger Hohlraum ausgebildet ist, dessen Durchmesser ungefähr dem Durchmesser des Luerkonus entspricht und/oder dessen axiale Ausdehnung ungefähr der Wandstärke des kegelstumpfförmigen Abschnitts entspricht. Die Erfinder haben festgestellt, dass durch die ringförmige Aussparung die Bruchresistenz der Spritze gesteigert werden kann, vermutlich da die axiale Kraftübertragung vom Luerkonus auf den kegelstumpfförmigen Abschnitt des Spritzenkörpers reduziert werden kann. Während bei einigen im Markt eingesetzten Konnektoren hohen axiale Druckkräfte auf den Luerkonus aufgebaut werden, die zu Rissen in dem kegelstumpfförmigen Abschnitt führen, treten derartige Risse durch den zylinderförmigen Hohlraum deutlich seltener auf.

In einer bevorzugten Ausführungsform weist die Spritze sowohl eine ringförmige Erhebung und eine ringförmige Aussparung gemäß den vorherigen Ausführungsformen auf, die so zueinander angeordnet sind, dass der Luerkonus im Wesentlichen über die ringförmige Erhebung mit dem kegelstumpfförmigen Abschnitt verbunden ist. Dadurch können zum einen die axialen Kräfte vom Luerkonus auf den kegelstumpfförmigen Abschnitt des Spritzenkörpers weiter reduziert werden, zum anderen weist die Spritze in einem Teilkörper, welcher aus Luerkonus und ringförmiger Erhebung besteht, eine relativ konstante Wandstärke auf. Das wirkt sich positiv auf das Spritzgussverhalten aus, da Stellen mit lokal größerer Wandstärke zu Problemen wie Einfallstellen und inneren Spannungen führen können, welche die Festigkeit der Spritze verringern können. Ferner kann durch die Vermeidung einer lokal größeren Wandstärke eine geringe Abkühlzeit und damit eine geringe Entformzeit erreicht werden.

Bevorzugt beträgt die Wandstärke im Bereich aus der ringförmigen Erhebung und dem daran anschließenden Konus gebildeten Teilkörper zwischen 50 % und 200 % der Wandstärke des Luerkonus und mehr bevorzugt zwischen 75 % und 125 %. Dabei ist die Wandstärke des Luerkonus, welche typischerweise zum Spritzenkörper hin geringfügig zunimmt, die Wandstärke in einem Spritzenkörper-nahen Abschnitt des Luerkonus.

In einer bevorzugten Ausführungsform sind die Übergänge der Kanten an dem Übergang des kegelstumpfförmigen Abschnitts zum Luerkonus, an dem Luerkonus, an dem Übergang vom Luerkonus zu der ringförmigen Erhebung und/oder an der ringförmigen Erhebung verrundet ausgebildet mit einem Radius von mindestens 0,1 mm und bevorzugt 0,15 mm bis 1,0 mm. Spannungsspitzen in den Übergangsbereichen können so reduziert werden und Risseinläufer in den Übergangskanten vermieden oder reduziert werden.

In einer bevorzugten Ausführungsform erstreckt sich das Innengewinde des Luerkragens bis zur Bodenfläche. Im Gegensatz zu einem Innengewinde, welches oberhalb der Bodenfläche endet, lässt sich ein bis zur Bodenfläche durchgängiges Innengewinde im Spritzguss mit Hilfe eines Schraubkerns fertigen, welcher zur Entformung nach dem Spritzguss ausgeschraubt wird. Eine Herstellung im Spritzgussverfahren ist also ohne weiteres möglich.

In einer besonders bevorzugten Ausführungsform beträgt der axiale Überstand des Luerkonus über den Luerkragen mindestens 2,2 mm und bevorzugt 2,3 mm. Dadurch kann die Gefahr eines zu tiefen Einschraubens eines Konnektors reduziert werden.

In einer bevorzugten Ausführungsform weist der Spritzenkörper ferner einen Flansch auf, welcher bevorzugt einstückig mit dem Spritzenkörper ausgebildet ist. Der Flansch kann zylindersymmetrisch oder nicht zylindersymmetrisch ausgebildet sein, um verschiedene drehbare oder auch im Drehwinkel fixierte Fingerflansche oder Backstop-Einrichtungen zu befestigen. Sogenannte Backstop-Einrichtungen verhindern das Ausziehen eines Stopfens und / oder einer Kolbenstange aus der Spritze.

In einer besonders bevorzugten Ausführungsform umfasst die Spritze ferner einen Stopfen und / oder eine Kolbenstange, welche gemäß Stand der Technik ausgebildet sein können. Zur Befestigung der Kolbenstange am Stopfen weist der Stopfen üblicherweise eine Aussparung auf, die mit einem Innengewinde versehen sein kann, in welches der Stopfen eingeschraubt wird.

Im Bereich der Erfindung liegt zudem auch ein pharmazeutisches Produkt. Es umfasst eine Ausführungsform einer Spritze, wie sie vorstehend beschrieben ist. Die Spritze ist mit einer medizinischen Flüssigkeit befüllt, die vorzugsweise einen Wirkstoff enthält. Eine medizinische Flüssigkeit ist zum Beispiel eine intravenös zu verabreichende Flüssigkeit. Beispiele für eine intravenös zu verabreichende Flüssigkeit sind eine Kochsalzlösung, eine Glukoselösung, eine Nährstofflösung für die parenterale Ernährung, eine Emulsion oder dergleichen. Die Wirkstoffe können gelöst und/oder dispergiert in der medizinischen Flüssigkeit vorliegen. Ein Beispiel für eine Emulsion stellt eine Propofol enthaltende Emulsion dar. Propofol wird beschrieben durch den chemischen Namen 2,6-Diisopropylphenol (IUAPC). Insbesondere für Propofol enthaltende Emulsionen hat sich die erfindungsgemäße Spritze als vorteilhaft erwiesen. Eine derartig befüllte Spritze besitzt selbst bei einem nachgeschalteten Autoklavieren eine verbesserte Stabilität.

Die Erfindung wird im Folgenden anhand von Figuren näher erläutert.

### Figurenbeschreibung

Fig. 1: Stand der Technik, perspektivische Schnitt-Ansicht eines Luer-Lock-Anschluss mit eingeschraubtem Konnektor
Fig. 2: Stand der Technik, Konnektoren gemäß ISO 594-2
Fig. 3: Längsschnitt durch eine erfindungsgemäße Spritze
Fig. 4: perspektivische Schnitt-Ansicht des Luer-Lock-Anschlusses einer erfindungsgemä-βen Spritze mit eingeschraubtem Konnektor
Fig. 5: Horizontaler Querschnitt durch Luer-Lock-Anschluss einer erfindungsgemäßen Spritze
Fig. 6: Horizontaler Querschnitt durch eine Verstärkungsrippe

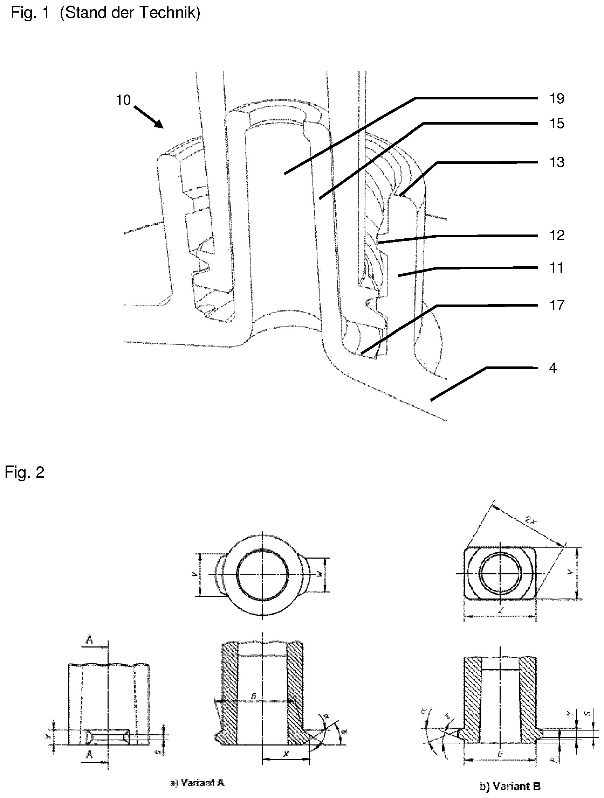

Figur 1 zeigt eine perspektivische Schnitt-Ansicht eines Luer-Lock-Anschluss mit eingeschraubtem Konnektor gemäß Stand der Technik. Auf einem kegelstumpfförmigen Abschnitt (4) des Spritzenkörpers befindet sich mittig der Luer-Konus (15) mit dem Durchlasskanal (19). Um den Luerkonus (15) ist der Luerkragen (11) mit einem Innengewinde (12) angeordnet. Zwischen Luerkonus (15) und Luerkragen (19) ist eine Bodenfläche (17) angeordnet, welche plan ausgebildet ist. Die Wandstärke des Spritzenkörpers im Bereich der Bodenfläche (17) entspricht ungefähr der Wandstärke des Spritzenkörpers im kegelstumpfförmigen Abschnitts (4).

Figur 2 zeigt zwei verschiedene Varianten A und B von Konnektoren gemäß ISO 594-2 zum Anschließen an einen Luer-Lock-Anschluss. Für Variante A ist ein Längsquerschnitt sowie eine Seitenansicht und eine Ansicht von oben dargestellt. Der Konnektor weist kein durchgängiges Außengewinde auf, sondern lediglich zwei sich gegenüberliegende Abschnitte eines Außengewindes, welche in einem unteren Abschnitt des Konnektors angeordnet sind und welche in das Innengewinde (12) des Luerkragens gemäß Figur 1 eingreifen können. Für Variante B ist ein Längsquerschnitt sowie eine Ansicht von oben dargestellt. Der Konnektor weist ebenfalls kein durchgängiges Außengewinde auf, sondern lediglich vier Abschnitte eines Außengewindes, welche in einem unteren Abschnitt des Konnektors angeordnet sind und welche in das Innengewinde (12) des Luerkragens gemäß Figur 1 eingreifen können.

Figur 3 zeigt einen Längsschnitt durch eine erfindungsgemäße Spritze (1), welche einen Spritzenkörper (2) mit einem zylindrischen Abschnitt (3) und einem kegelstumpfförmigen Abschnitt (4) aufweist, in welchem sich der Spritzenkörper (1) zum Durchlasskanal (19) hin konisch verjüngt. Am proximalen Ende ist ein Flansch (5) angeordnet. Der Luer-Lock-Anschluss (10) umfasst einen Luerkonus (15) und einen Luerkragen (11). Die Spritze besteht aus einem COC-Material und der Spritzenkörper weist im zylindrischen Bereich eine Wandstärke von ungefähr 1,5 mm auf. Die Innenseite des Spritzenkörpers weist eine Gleitmittelbeschichtung mit einem Silikonöl auf.

Figur 4 zeigt eine perspektivische Schnitt-Ansicht des Luer-Lock-Anschlusses (10) einer erfindungsgemäßen Spritze mit eingeschraubtem Konnektor, wobei der Querschnitt durch Verstärkungsrippen (18) läuft, welche an der Außenseite des Luerkragens (11) angeordnet sind.

Die Verstärkungsrippen (18) erstrecken sich vom kegelstumpfförmigen Abschnitt (4) ausgehend axial entlang der Außenwand des Luerkragens (11) und enden unterhalb des freien Endes des Luerkragens (11). Die Übergangsbereiche zwischen den Verstärkungsrippen (18) und dem kegelstumpfförmigen Abschnitt (4) sind stark verrundet ausgebildet mit einem Radius von ungefähr 1,0 mm und die Übergangsbereiche zwischen den Verstärkungsrippen (18) und dem zylindrischen Abschnitt des Luerkragens (11) sind ebenfalls verrundet ausgebildet mit einem Radius von ungefähr 0,5 mm.

Der Luer-Lock-Anschluss (10) umfasst ferner den Luerkonus (15) mit dem Durchlasskanal (19). Zwischen Luerkonus (15) und Luerkragen (11) ist eine Bodenfläche (17) ausgebildet. Das Innengewinde (12) des Luerkragens (11) erstreckt sich bis zur Bodenfläche (17). Am unteren Ende des Luerkonus (15) ist auf der Bodenfläche (15) eine ringförmige Erhebung (20) ausgebildet, welche in der Querschnittsansicht stufenförmig ausgebildet ist. Die ringförmige Erhebung (20) weist vom Niveau des tiefsten Punktes der Bodenfläche (17) aus gemessen eine axiale Höhe von ca. 1,2 mm auf und eine radiale Ausdehnung von 0,7 mm. Innerhalb des Spritzenkörpers (2) ist eine ringförmige Aussparung (21) ausgebildet, welche ungefähr in einem Schnittbereich des nach unten verlängerten Spritzenkonus (15) und des nach innen verlängerten kegelstumpfförmigen Abschnitts (4) angeordnet ist. Der Luerkonus (15) ist im Wesentlichen über die ringförmige Erhebung (20) mit dem kegelstumpfförmigen Abschnitt (4) des Spritzenkörpers verbunden. Die Wandstärke in dem Teilkörper bestehend aus ringförmiger Erhebung (20) und dem Luerkonus (15) ist relativ konstant. Aufgrund der ringförmigen Aussparung (21) entsteht im unteren Bereich des Durchlasskanals ein ungefähr zylinderförmiger Hohlraum (23), welcher eine Aufweitung des Durchlasskanals (19) in seinem unteren Bereich bildet. Die Übergangsbereiche auf der Außenseite des Spritzenkörpers zwischen Luer-Konus (15), ringförmiger Erhebung (20), Bodenfläche (17) und Luerkragen sind verrundet ausgebildet mit Radien im Bereich von 0,15 mm bis 0,5 mm. Die Übergangsbereiche an der Innenseite des Spritzenkörpers zwischen Luer-Konus (15), ringförmiger Aussparung (21), und kegelstumpfförmigen Abschnitt (4) sind ebenfalls verrundet ausgebildet mit einem Radius von ungefähr 0,2 mm.

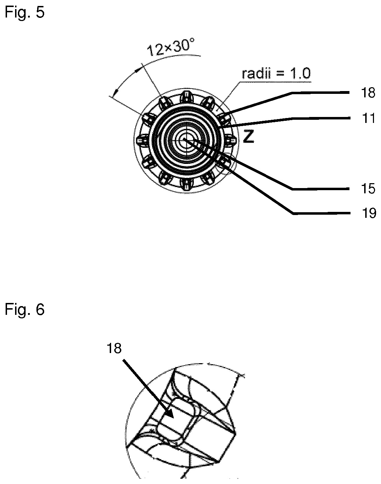

Figur 5 zeigt einen horizontalen Querschnitt durch den Luer-Lock-Anschluss einer erfindungsgemäßen Spritze. Auf der Außenseite des Luerkragens (11) sind 12 Verstärkungsrippen (18) in einem Winkelabstand von jeweils 30° um den Luerkragen angeordnet. Die Verstärkungsrippen weisen an ihrer Basis ungefähr einen rechteckigen Querschnitt auf mit einer Breite von ungefähr 0,8 mm auf und einer radialen Abmessung von ungefähr 0,6 mm. Dabei ist unter der Basis ein unterer Abschnitt der Verstärkungsrippen zu verstehen, in welchen die Übergangsradien zum kegelstumpfförmigen Abschnitt (4) jedoch nicht mehr hineinreichen und welcher in Längsrichtung der Spritze ungefähr auf Höhe der ringförmigen Erhebung (20) liegt.

Figur 6 zeigt eine Ausschnittvergrößerung von Figur 5. Es ist zu erkennen, dass die Verstärkungsrippen (18) an ihrem Fuß verrundete Übergangsbereiche zum kegelstumpfförmigen Abschnitt (4) aufweisen, sowie auch an ihrem oberen Ende eine verrundete Kante mit einem Radius von ungefähr 0,2 mm aufweisen.

### Bezugszeichenliste

- 1: Spritze
- 2: Spritzenkörper
- 3: zylindrischer Abschnitt
- 4: kegelstumpfförmiger Abschnitt
- 5: Fingerflansch
- 10: Luer-Lock-Anschluss
- 11: Luerkragen
- 12: Innengewinde
- 13: Oberkante des Luerkragens
- 15: Luerkonus
- 17: Bodenfläche
- 18: Verstärkungsrippen
- 19: Durchlasskanal
- 20: ringförmige Erhebung
- 21: ringförmige Aussparung
- 23: zylinderförmiger Hohlraum
- 30: Konnektor

## Patentansprüche

1. Spritze (1) umfassend:
- einen Spritzenkörper (2), welcher einen zylindrischen Abschnitt (3) und einen daran anschließenden kegelstumpfförmigen Abschnitt (4) aufweist, und
- einen Luer-Lock-Anschluss (10), welcher einen Luerkragen (11) mit einem Innengewinde (12) und einen Luerkonus (15) mit einem Durchlasskanal (19) aufweist, wobei zwischen Luerkragen (11) und Luerkonus (15) eine Bodenfläche (17) ausgebildet ist,
wobei Spritzenkörper (2) und Luer-Lock-Anschluss (10) einstückig ausgebildet sind und aus einem zyklischen Olefinharz bestehen;
wobei der Luerkragen (11) auf seiner Außenseite axial verlaufende Verstärkungsrippen (18) aufweist, und
wobei die Verstärkungsrippen (18) an ihrer dem kegelstumpfförmigen Abschnitt (4) zugewandten Basis eine radiale Ausdehnung zwischen 0,5 mm und 2,5 mm aufweisen und eine laterale Ausdehnung zwischen 0,5 mm und 2,0 mm aufweisen, und/oder
wobei der Übergang der Verstärkungsrippen (18) zum Luerkragen (11) und/oder zum kegelstumpfförmigen Abschnitt (4) verrundet ausgebildet ist mit einem Radius von mindestens 0,3 mm.

2. Spritze nach Anspruch 1, wobei der Luerkragen (11) zwischen 4 und 24, bevorzugt zwischen 8 und 16 Verstärkungsrippen (18) aufweist.

3. Spritze nach einem der vorhergehenden Ansprüche, wobei die Verstärkungsrippen (18) sich vom kegelstumpfförmigen Abschnitt (4) ausgehend axial bis mindestens zur halben Höhe des Luerkragens (11) und bevorzugt bis zur Oberkante (13) des Luerkragens (11) erstrecken.

4. Spritze nach einem der vorhergehenden Ansprüche, wobei der Übergang der Verstärkungsrippen (18) zum Luerkragen (11) und/oder zum kegelstumpfförmigen Abschnitt (4) verrundet ausgebildet ist mit einem Radius von 0,5 bis 1,5 mm.

5. Spritze nach einem der vorhergehenden Ansprüche, wobei auf der Bodenfläche (18) und an der Außenseite des Luerkonus (15) eine ringförmige Erhebung (20) angeordnet ist, welche so ausgebildet ist, dass sie als Anschlag für einen einschraubbaren Konnektor (50) wirkt.

6. Spritze nach einem der vorhergehenden Ansprüche, mit einer ringförmigen Aussparung (21), welche radial in der Verlängerung des kegelstumpfförmigen Abschnittes (4) und axial unterhalb der Wandung des Spritzenkonus (15) liegt, so dass zwischen Durchlasskanal (19) und kegelstumpfförmigen Abschnitt (4) ein zylinderförmiger Hohlraum (22) ausgebildet ist, dessen Durchmesser ungefähr dem Durchmesser des Luerkonus entspricht und/oder dessen axiale Ausdehnung ungefähr der Wandstärke des kegelstumpfförmigen Abschnitts (4) entspricht.

7. Spritze nach den vorhergehenden Ansprüchen 5 und 6, wobei die ringförmige Erhebung (20) und die ringförmige Aussparung (21) so zueinander angeordnet sind, dass der Luerkonus (15) im Wesentlichen über die ringförmige Erhebung (20) mit dem kegelstumpfförmigen Abschnitt (4) verbunden ist.

8. Spritze nach Anspruch 7, wobei die Wandstärke im Bereich aus der ringförmigen Erhebung und dem daran anschließenden Konus gebildeten Teilkörper zwischen 50% und 200% der Wandstärke des Luerkonus (15) beträgt.

9. Spritze nach Anspruch 7, wobei die Übergänge der Kanten an dem Übergang des kegelstumpfförmigen Abschnitts (4) zum Luerkonus (15), an dem Luerkonus (15), an dem Übergang vom Luerkonus (15) zu der ringförmigen Erhebung (20) und/oder an der ringförmigen Erhebung (20) verrundet ausgebildet sind mit einem Radius von mindestens 0,1 mm und bevorzugt 0,15 bis 1.0 mm.

10. Spritze nach einem der vorhergehenden Ansprüche, wobei sich das Innengewinde (12) des Luerkragens (11) bis zur Bodenfläche (17) erstreckt.

11. Spritze nach einem der vorhergehenden Ansprüche, wobei der axiale Überstand des Luerkonus (15) über den Luerkragen (11) mindestens 2,2 mm beträgt.

12. Spritze nach einem der vorhergehenden Ansprüche, wobei der Spritzenkörper (2) ferner einen Fingerflansch (5) aufweist, welcher bevorzugt einstückig mit dem Spritzenkörper (2) ausgebildet ist.

13. Spritze nach einem der vorhergehenden Ansprüche, umfassend einen Stopfen und/oder eine Kolbenstange.

14. Pharmazeutisches Produkt umfassend eine Spritze nach einem der vorstehenden Ansprüche, wobei die Spritze mit einer medizinischen, vorzugsweise einen Wirkstoff enthaltenden, Flüssigkeit befüllt ist.

## Claims

1. Syringe (1) comprising:
- a syringe body (2), which has a cylindrical portion (3) and, adjoining the latter, a frustoconical portion (4), and
- a Luer lock connector (10), which has a Luer collar (11) with an inner thread (12) and a Luer cone (15) with a through-channel (19), wherein a bottom surface (17) is formed between Luer collar (11) and Luer cone (15),
wherein the syringe body (2) and the Luer lock connector (10) are formed in one piece and are made of a cyclic olefin resin;
wherein the Luer collar (11) has axially extending reinforcing ribs (18) on its outer face; and wherein the reinforcing ribs (18) have a radial extent of between 0.5 mm and 2.5 mm at their base facing the frustoconical portion (4) and have a lateral extent of between 0.5 mm and 2.0 mm; and/or wherein the transition of the reinforcing ribs (18) to the Luer collar (11) and/or to the frustoconical portion (4) is rounded, with a radius of at least 0.3 mm.

2. Syringe according to Claim 1, wherein the Luer collar (11) has between 4 and 24, preferably between 8 and 16 reinforcing ribs (18).

3. Syringe according to one of the preceding claims, wherein the reinforcing ribs (18), starting from the frustoconical portion (4), extend axially at least as far as half the height of the Luer collar (11) and preferably as far as the upper edge (13) of the Luer collar (11).

4. Syringe according to one of the preceding claims, wherein the transition of the reinforcing ribs (18) to the Luer collar (11) and/or to the frustoconical portion (4) is rounded, with a radius of 0.5 to 1.5 mm.

5. Syringe according to one of the preceding claims, wherein an annular elevation (20) is arranged on the bottom surface (18) and on the outer face of the Luer cone (15) and is designed such that it acts as an abutment for a screw-in attachment (50).

6. Syringe according to one of the preceding claims, with an annular recess (21) which lies radially in the continuation of the frustoconical portion (4) and axially below the wall of the syringe cone (15), such that a cylindrical cavity (22) is formed between through-channel (19) and frustoconical portion (4), the diameter of which cylindrical cavity (22) corresponds approximately to the diameter of the Luer cone and/or the axial extent of which corresponds approximately to the wall thickness of the frustoconical portion (4).

7. Syringe according to Claims 5 and 6, wherein the annular elevation (20) and the annular recess (21) are arranged in relation to each other such that the Luer cone (15) is connected to the frustoconical portion (4) substantially via the annular elevation (20) .

8. Syringe according to Claim 7, wherein the wall thickness in the body region formed of the annular elevation and of the adjoining cone is between 50% and 200% of the wall thickness of the Luer cone (15) .

9. Syringe according to Claim 7, wherein the transitions of the edges at the transition of the frustoconical portion (4) to the Luer cone (15), at the Luer cone (15), at the transition from the Luer cone (15) to the annular elevation (20) and/or at the annular elevation (20) are rounded, with a radius of at least 0.1 mm and preferably of 0.15 mm to 1.0 mm.

10. Syringe according to one of the preceding claims, wherein the inner thread (12) of the Luer collar (11) extends as far as the bottom surface (17).

11. Syringe according to one of the preceding claims, wherein the axial protrusion of the Luer cone (15) above the Luer collar (11) is at least 2.2 mm.

12. Syringe according to one of the preceding claims, wherein the syringe body (2) moreover has a finger flange (5), which is preferably configured in one piece with the syringe body (2).

13. Syringe according to one of the preceding claims, comprising a stopper and/or a piston rod.

14. Pharmaceutical product comprising a syringe according to one of the preceding claims, wherein the syringe is filled with a medical liquid, preferably a medical liquid containing an active substance.

## Revendications

1. Seringue (1), comprenant :
- un corps de seringue (2) qui présente une partie cylindrique (3) et une partie tronconique (4) adjacente à celle-ci, et
- un embout Luer-Lock (10) qui présente une collerette Luer (11) munie d'un taraudage (12) et un cône Luer (15) muni d'un canal de passage (19), une surface de fond (17) étant réalisée entre la collerette Luer (11) et le cône Luer (15),
dans laquelle le corps de seringue (2) et l'embout Luer-Lock (10) sont réalisés d'un seul tenant et sont composés d'une résine d'oléfine cyclique ;
dans laquelle la collerette Luer (11) présente des nervures de renforcement (18) s'étendant axialement sur sa face extérieure ; et
dans laquelle les nervures de renforcement (18) présentent sur leur base tournée vers la partie tronconique (4) une étendue radiale comprise entre 0,5 mm et 2,5 mm et une étendue latérale comprise entre 0,5 mm et 2,0 mm ; et/ou
dans laquelle la transition des nervures de renforcement (18) à la collerette Luer (11) et/ou à la partie tronconique (4) est réalisée de manière arrondie avec un rayon d'au moins 0,3 mm.

2. Seringue selon la revendication 1, dans laquelle la collerette Luer (11) présente entre 4 et 24, de préférence entre 8 et 16, nervures de renforcement (18) .

3. Seringue selon l'une quelconque des revendications précédentes, dans laquelle les nervures de renforcement (18) s'étendent à partir de la partie tronconique (4) axialement à au moins mi-hauteur de la collerette Luer (11) et de préférence jusqu'au bord supérieur (13) de la collerette Luer (11).

4. Seringue selon l'une quelconque des revendications précédentes, dans laquelle la transition des nervures de renforcement (18) à la collerette Luer (11) et/ou à la partie tronconique (4) est réalisée de manière arrondie avec un rayon de 0,5 à 1,5 mm.

5. Seringue selon l'une quelconque des revendications précédentes, dans laquelle, sur la surface de fond (18) et sur la face extérieure du cône Luer (15), un relief annulaire (20) est disposé qui est réalisé de façon à agir comme une butée pour un connecteur à visser (50).

6. Seringue selon l'une quelconque des revendications précédentes, comprenant un évidement annulaire (21) qui se trouve radialement dans le prolongement de la partie tronconique (4) et axialement au-dessous de la paroi du cône de seringue (15) de sorte qu'entre le canal de passage (19) et la partie tronconique (4) une cavité cylindrique (22) est réalisée dont le diamètre correspond approximativement au diamètre du cône Luer et/ou dont l'étendue axiale correspond approximativement à l'épaisseur de paroi de la partie tronconique (4).

7. Seringue selon les revendications précédentes 5 et 6, dans laquelle le relief annulaire (20) et l'évidement annulaire (21) sont disposés l'un par rapport à l'autre de telle sorte que le cône de Luer (15) est relié à la partie tronconique (4) substantiellement par l'intermédiaire du relief annulaire (20).

8. Seringue selon la revendication 7, dans laquelle l'épaisseur de paroi dans la zone du corps partiel formé par le relief annulaire et le cône adjacent à celui-ci est comprise entre 50 % et 200 % de l'épaisseur de paroi du cône Luer (15).

9. Seringue selon la revendication 7, dans laquelle les transitions des bords au niveau de la transition de la partie tronconique (4) au cône Luer (15), au niveau du cône Luer (15), au niveau de la transition du cône Luer (15) au relief annulaire (20) et/ou au niveau du relief annulaire (20) sont réalisées de manière arrondie avec un rayon d'au moins 0,1 mm et de préférence de 0,15 à 1,0 mm.

10. Seringue selon l'une quelconque des revendications précédentes, dans laquelle le taraudage (12) de la collerette Luer (11) s'étend jusqu'à la surface de fond (17) .

11. Seringue selon l'une quelconque des revendications précédentes, dans laquelle la saillie axiale du cône Luer (15) au-delà de la collerette Luer (11) mesure au moins 2,2 mm.

12. Seringue selon l'une quelconque des revendications précédentes, dans laquelle le corps de seringue (2) présente en outre une bride de préhension (5) qui est réalisée de préférence d'un seul tenant avec le corps de seringue (2).

13. Seringue selon l'une quelconque des revendications précédentes, comprenant un bouchon et/ou une tige de piston.

14. Produit pharmaceutique comprenant une seringue selon l'une quelconque des revendications précédentes, dans lequel la seringue est remplie d'un liquide médical contenant de préférence un principe actif.
